(19) Europäisches Patentamt
European Patent Office
Office européen des brevets

(11) Publication number : 0 297 019 B1

# (12) EUROPEAN PATENT SPECIFICATION

(45) Date of publication of patent specification : 30.10.91 Bulletin 91/44

(51) Int. Cl.$^5$ : C07D 277/48, A61K 31/425

(21) Application number : 88500054.7

(22) Date of filing : 01.06.88

## (54) Famotidine polymorphic form and preparation thereof.

The file contains technical information submitted after the application was filed and not included in this specification

(30) Priority : 22.06.87 ES 8702020
29.10.87 ES 8703326
23.03.88 ES 8800888

(43) Date of publication of application : 28.12.88 Bulletin 88/52

(45) Publication of the grant of the patent : 30.10.91 Bulletin 91/44

(84) Designated Contracting States : AT BE CH DE ES FR GB GR IT LI LU NL SE

(56) References cited :
EP-A- 0 128 736
EP-A- 0 256 747
US-A- 4 496 737

(73) Proprietor : CENTRO MARGA PARA LA INVESTIGACION S.A.
Muntaner 212, 5o-510
E-08036 Barcelona (ES)

(72) Inventor : Ballester-Rodes, Montserrat
Marqués de Santa Ana 14, 2a
E-08023-Barcelona (ES)
Inventor : Palomo-Nicolau, Francisco Eugenio
Dos de Mayo 34, 2o-1a
E-08190-Sant Cugat del Vallés (Barcel.) (ES)
Inventor : Palomo-Coll, Antonio Luis
Escuelas Pias 18, 5o-1a
E-08017-Barcelona (ES)

(74) Representative : Ballester Rodés, Albert
Muntaner 212, 5o- 508
E-08036 Barcelona (ES)

EP 0 297 019 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Famotidine is a generic term used to identify the chemical compound N-sulfamoyl-3-[(2-guanidinothiazol-4-yl)methylthio]propionamidine :

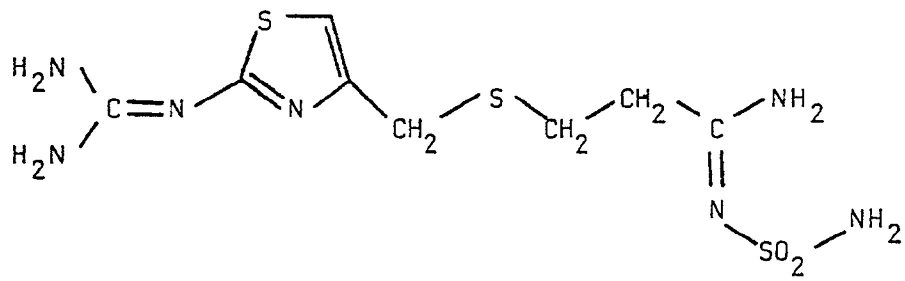

This compound inhibits the secretion of hydrochloric acid and pepsin, being used in the treatment of the gastric and duodenal ulcers, as in the Zolliger-Ellison syndrome, and other diseases of the upper digestive track.

The Famotidine is a $H_2$-receptor antagonist. In GB-A-2055800, a process is described where 3-[(2-guanidinothiazol-4-yl)methylthio]propionimidate is made to react with sulfamide, and by evaporation a residue is obtained which is purified in solution by chromatography through silicagel, yielding a product with m.p. 163-164°C. In US-A-4.496.737, the aqueous methanol solution, resulting from the reaction of N-sulfamylacrylamidine with 2-guanidinothiazol-4-methylthiol generated "in situ", is evaporated, and the residue is dissolved and passed through silicagel yielding a product with m.p. 160°C. In either case, the eluent is a methanol-chloroform mixture.

EP-A-0128736, describes a method to obtain Famotidine melting at 163-164°C from its acetate in aqueous solution, by hydrolysis and precipitation with aqueous sodium hydroxide.

The scarce solubility of Famotidine in methanol (0.5-1.0%) makes industrially impractical its purification by recrystallization from this solvent, even adding chloroform. By recrystallization from other organic solvents, such as dimethylformamide, dimethylacetamide, dimethylsulfoxide or acetonitrile, by dilution either with water or, preferably, with dichlorometane or chloroform, it results a product of variable melting point within the 164-176°C interval. Microscopic observation reveals the existence of conglomerates.

The Famotidine hydrochloride is scarcely soluble in water. An aqueous suspension at pH 7.0-7.5 yields Famotidine as granular concretions with a melting point interval of 158-172°C.

The variations in melting point and infrared spectrum reveal the presence of polymorphic forms. The different physical aspects are related to the crystal structure, which in turn is associated to purity and bulk density. Consequently, it was important to achieve the most favorable polymorphic form for pharmaceutical use.

In this connection it is mentioned that EP-A-0256747 refers to a process for the preparation of polymorphic forms of Famotidine.

The basis of this invention is the discovery of a polymorphic Famotidine CMI-BB which is most suitable for pharmaceutical and medical purposes. Three pure Famotidine polymorphic forms are obtainable : CMI-AA, m.p. 167-170°C, bulk density 0.70-0.80 g/ml ; CMI-BA, m.p. 158-161°C, bulk density 0.70-0.80 g/ml ; and CMI-BB, m.p. 157-161°C, bulk density 0.20-0.30 g/ml. The polymorphs CMI-BA and CMI-BB have identical IR and UV spectra. Nevertheless, the X-ray diffraction patterns are significantly different. They all are obtained from Famotidine hydrochloride by precipitation or crystallization with a tertiary organic base. These polymorphs and their mixtures may be converted into the CMI-BB polymorph, this being favored by seeding. These polymorphs differ also in their physico-chemical properties and bioactive behavior.

The Famotidine polymorphs are grouped into high- and low-density forms. Both are constituted by acicular crystals. The "high-density" forms have a bulk density mainly within the range 0.70-0.80 g/ml, the crystals being large. The bulk density of the "low density" forms is mainly in the 0.20-0.30 g/ml range, the crystals being small. Intermediate density values would correspond to polymorph mixtures, including the high-melting polymorph.

These materials here described do not present static charges and their characteristics make possible the direct use in some operations of pharmaceutical formulations.

The low density CMI-BB polymorph, described and claimed herein, is characterised by a bulk density comprised between 0.20 and 0.30 g/ml, a melting range within 157-161°C possesses the following unique X-Ray-diffraction properties at $\lambda = 1.5405$ Å, using a radiation source of Cu, 40 kw and 20 ma (Siemens Kristallofex 810 Interface DACO-MP equipment) :

| d: | | $I/I_1$ |
|---|---|---|
| 14.68 | ..... | 0.28 |
| 7.62 | ..... | 0.58 |
| 7.15 | ..... | 0.14 |
| 5.87 | ..... | 0.12 |
| 5.61 | ..... | 0.41 |
| 5.03 | ..... | 0.20 |
| 4.92 | ..... | 0.38 |
| 4.57 | ..... | 0.41 |
| 4.42 | ..... | 1.00 |
| 4.31 | ..... | 0.20 |
| 4.25 | ..... | 0.64 |
| 3.95 | ..... | 0.52 |
| 3.89 | ..... | 0.41 |
| 3.81 | ..... | 0.32 |
| 3.69 | ..... | 0.68 |
| 3.61 | ..... | 0.33 |
| 3.47 | ..... | 0.09 |
| 3.40 | ..... | 0.19 |
| 3.37 | ..... | 0.20 |
| 3.27 | ..... | 0.21 |
| 3.18 | ..... | 0.06 |
| 3.09 | ..... | 0.08 |
| 2.95 | ..... | 0.28 |
| 2.91 | ..... | 0.12 |
| 2.85 | ..... | 0.13 |
| 2.77 | ..... | 0.36 |
| 2.72 | ..... | 0.05 |
| 2.62 | ..... | 0.06 |
| 2.53 | ..... | 0.19 |
| 2.50 | ..... | 0.14 |

| | |
|---|---|
| 2.46 ..... | 0.07 |
| 2.42 ..... | 0.07 |
| 2.40 ..... | 0.09 |
| 2.29 ..... | 0.10 |
| 2.23 ..... | 0.06 |
| 2.20 ..... | 0.11 |
| 2.17 ..... | 0.07 |
| 2.15 ..... | 0.12 |
| 2.12 ..... | 0.06 |
| 2.11 ..... | 0.11 |
| 2.03 ..... | 0.08 |
| 1.97 ..... | 0.07 |
| 1.85 ..... | 0.05 |
| 1.83 ..... | 0.06 |
| 1.79 ..... | 0.06 |
| 1.76 ..... | 0.06 |
| 1.74 ..... | 0.07 |
| 1.70 ..... | 0.05 |
| 1.61 ..... | 0.05 |
| 1.60 ..... | 0.13 |

The application EP-A-0256747 refers to the metastable, low melting point polymorph B, with bulk density values of 0.34 g/ml and 0.50 g/ml (page 5, lines 8-11). This indicates therefore that such a polymorph is actually a mixture of high and low density polymorphs.

The CMI-BB polymorph claimed in this invention can be obtained from any Famotidine, regardless of its method of synthesis and its infrared spectrum, provided that its melting point is within the interval 160-180°C.

The process here claimed starts with the preparation of Famotidine hydrochloride, hydrobromide, sulfate, nitrate or phosphate. It consists in the following steps :
(1) The Famotidine is suspended in methanol. (2) About a stoichiometric amount of an acid which is HCl, HBr, $H_2SO_4$, $HNO_3$ or $H_3PO_4$, enough to obtain a clear solution, is added. (3) At a temperature between 0-25°C, a tertiary organic base, the salt of which in that acid is soluble in methanol or in its mixture with dichloromethane or chloroform, is quickly added with stirring. (4) The temperature is increased to 50-55°C until a solution is obtained. (5) The resulting mass is diluted with dichloromethane or chloroform, which causes a quick crystallization. (6) The mass is cooled down to 0-10°C. (7) The crystalline mass is separated by filtration, decantation or other procedure at a temperature of 0-15°C.

It is emphasized that a slow addition of the organic base leads to a beginning of the crystallization at a pH near 2.0 and ending at 6.5-7.0 which affords an undesirable product melting at 186-188°C. If the conversion to the proper polymorph is attempted by suspending it with stirring in water at pH 7.0-7.5, a polymorphic mixture melting at 165-173°C is obtained instead. Therefore, to obtain the low-density polymorph, a quick addition of the organic base should be effected.

Mixtures of polymorphs melting at 172-174°C or 169-172°C are obtained from an acetonitrile or dimethylformamide solution of Famotidine, by addition of a relatively small amount of water, and then cooling down to 0-10°C. Mixtures of polymorphs melting from 150 to 168°C are obtained when a solution of Famotidine hydrochloride in methanol is first diluted with dichloromethane and then brought to a slightly alkaline pH with an organic base.

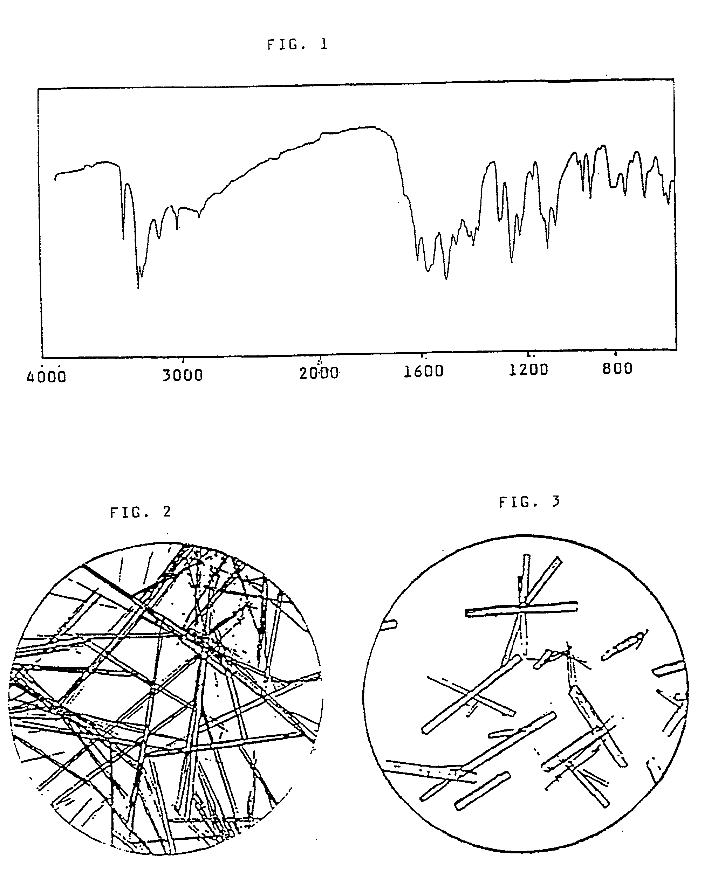

The polymorph CMI-BB (Figure 1) is e.g. obtained from the solutions of Famotidine hydrochloride in methanol, brought to a slightly alkaline pH. The precipitate formed is redissolved by heating, diluting next with dichloromethane or chloroform, and keeping it for some time at 10-15°C.

For the purposes of this invention useful tertiary organic bases are triethylamine, N-ethylpiperidine, N-methylmorpholine, N-tripropylamine, and the bicyclic amidines 1,8-diazabicyclo[5.4.0]undec-7-ene (DBU), 1,5-diazabicyclo[4.3.0]non-5-ene (DBN) ; in general, those tertiary organic bases the hydrochloride salts of which are soluble in methanol or in its mixture with dichlorometane or chlorofrom.

Polymorph CMI-BB shows higher chemical purity, better physical and chemical stability, and, surprisingly, greater bioactivity than those of the other polymorphic forms.

The CMI-BB withstands a pressure of 5-10 tons, being transformed into a white powder without experimenting variation in the melting point. If the crystallization has not been controlled rigorously, the low-density product might show a slight melting point increment (table 1, CMI-MB).

The preparation of CMI-BB polymorph is significantly improved by seeding, preferably between 30-50°C. Dilution with dichloromethane or chloroform, is important as far as reaching the bulk density which is characteristic of CMI-BB polymorph. Dilution is effected before seeding.

Methanol is selected as the solvent to ensure an adequate solubility of the organic base salt.

Examples 1, 2, 4, 6, 8 and 11 of the present invention refer to mixtures or high density polymorphs, which are useful for the subsequent conversion into the CMI-BB polymorph. Examples 9 and 10 are not illustrative, nevertheless they disclose operative methods showing the effect of pH in the Famotidine salt solution, and the fast crystallization.

The Famotidine polymorph CMI-BB, obtained by the fast crystallization procedure, has been submitted to a study for bioactivity, versus the other forms.

The study of the antiulcerous activity of the polymorphs has been realized in lots of male Wistar rats, with weights comprised between 202 and 230 g, distributed in six groups of treatment, as indicated in the table 1.

The polymorphs were suspended in a solution of carboxymethylcellulose (0.5%) in distilled water. They were administered in the ratio of 10 ml/kg, p.o. Indomethacin, the ulcerogenic agent, was applied 30 min. after the treatments (30 mg/10 ml/kg, p.o.), and the animals were sacrificed 6 hours later. The stomachs were extracted and the gastric lesions found were evaluated in millimeters of length (mm).

The antiulcerous activity of the tested polymorphs, was evaluated by inhibition-percentage calculated from the average length in each treated group and the average in the control group. The results obtained in the different groups were compared statistically by means of correlation analysis and the Duncan-Kramer test. The results are summarized in table 2.

There are significant activity differences among the polymorphs, being most remarkable those between the extreme groups B and F (table 2).

The low-melting polymorph (C) showed a 30% higher activity than the high-melting one (F). Also, significant differences are found depending upon the relative bulk density (tables 1 and 2).

Therefore, polymorph CMI-BB is the best for a therapeutic use in Medicine and in pharmaceutical formulations, particularly those that do not modify its crystalline structure. The inorganic and organic salts, as well as the aminoacids in the relevant pharmaceutical composition should not alter its polymorphic form.

Table 1

| Group | Treatment | MP(°C) | BD (g/ml) | Dose (mg/kg ; p.o.) |
|---|---|---|---|---|
| A | Control | ----- | ----- | ----- |
| B | Cimetidine (Reference) | ----- | ----- | 100 |
| C | CMI-BB | 158-160 | 0.20 | 6 |
| D | CMI-MB | 160-163 | 0.28 | 6 |
| E | CMI-BA | 158-161 | 0.77 | 6 |
| F | CMI-AA | 167-170 | 0.78 | 6 |

## TABLE 2

FAMOTIDINE POLYMORPHS: ANTIULCER ACTIVITY

| GROUP | TREATMENT (Drug) | DOSE (mg/Kg;p.o.) | WEIGHT (g) (Med ± EE) | ULCERS (mm) (Med ± EE) | INHIBITION (% respect to control) |
|---|---|---|---|---|---|
| A | Control | .... | 218,7 ± 2,84 | 70,5 ± 7,89 | .... |
| B | Cimetidine[a] | 100 | 212,8 ± 2,02 | 7,0 ± 2,33 | 90,0 |
| C | CMI-BB | 6 | 212,6 ± 2,20 | 0 | 100,0 |
| D | CMI-MB | 6 | 218,4 ± 1,95 | 2,5 ± 1,12 | 96,4 |
| E | CMI-BA | 6 | 214,2 ± 1,94 | 13,2 ± 4,77 | 81,3 |
| F | CMI-AA | 6 | 211,8 ± 2,22 | 22,0 ± 5,17 | 68,8 |

Famotidines: CMI protocol. Average - standard error (n = 10). Statisticly significant differences $p < 0,05$ (Duncan-kramer test). (a) Reference.

Example 1

Polimorph mixture

A. To a suspension of Famotidine (3.0 g, m.p. 173-176°C) in water (15 ml), concentrated aqueous hydrochloric acid (1.86 ml, 35,0%) is added, yielding a thick white mass of the Famotidine hydrochloride. After cooling the mass on ice-water bath, the pH is adjusted to pH = 7.0-7.5 with triethylamine (2.5 ml), and stirred during 60 min. The white precipitate is collected by filtration, washed with water and with methanol, yielding, after drying, 2.03 g of Famotidine, which melts partly at 158°C, ending at 172°C. Microscopic observation shows a major formation of concretions. I.R. (KBr) $\nu cm^{-1}$ : 3400, 3300, 3220 (sh), 3100, 2880, 1685, 1650 (m) 1600 and 1550.

B. To the suspension of Famotidine (3.0 g, m.p. = 155-161°C in methanol (15.0 ml) concentrated aqueous hydrochlorid acid (1.86 ml, 35%) is added, resulting a colorless solution, temperature 33°C and pH = 1.0. The resulting mass is cooled down to 0-5°C (in about 10-15 min.) and triethylamine (2.5 ml) is added gradually, keeping pH = 1.8-2.0 during the precipitation. In the course of about 30 min., pH remains at 2.0, reaching finally the pH = 6.5. The precipitate is collected by filtration, washed with methanol, dichloromethane, and dried yielding 2.59 g of product with m.p. 186-188°C. I.R. (KBr) $\nu cm^{-1}$ : 3420 (sh, s), 3300, 3220 (m), 3170 (m), 3100 (m), 2980, 2890, 1675, 1650 (w), 1610 y 1560.

The solid obtained is suspended again in methanol and stirred during a time (15 min.) with trimethylamine at pH = 7.5, yielding after filtration, a mixture of polymorphs m.p. 165-173°C. The X-Ray-diffraction properties are markedly different from those corresponding to the forms constituted by high and low density prisms described later.

Example 2

High-density polymorph

To a stirred Famotidine suspension (100.0 g ; IR (KBr) $\nu$ $cm^{-1}$ : 3430 and 3380 (duplest, s), 3300 (s), 3220 (s), 1660 and 1635 (duplet, s), 1600 (s) and 1535 (s) in methanol (1500 ml), concentrated aqueous hydrochloric aced (35%, density 1.18 ; 60.0 ml, 70.8 cmol approximately) is added first, and next, to the resulting solution, triethylamine (100.0 mL ; 71.0 cmol). Raising of the temperature of the mass (interval 25-35°C), causes the formation of ε. precipitate. The mass is heated until it simmers (63°C). While hot, to the resulting solution dichloromethane (3000 mL) is gradually added, letting it stand at the temperature of 10-15°C during 3-4 hours.

The precipitate formed is collected by filtration, and washed with methanol and with dichloromethane, yielding 75.0 g of the title compound, m.p. 159-161°C, bulk density 0.55, infrared spectrum shown in Figure 1, crystals as in Figure 2.

Observation with a stereoscopic microscope (Kiowa SDZ-PL model, provided with zoom), shows an homogeneous mixture of long acicular white prisms. The melting point is uncorrected and taken with a binocular microscope (Reichert Thermovar).

From the mother liquors, 10.0 to 15.0 g more of Famotidine and the solvents are recovered.

Example 3

Low-density polymorph CMI-BB

Famotidine, IR(KBr) $\nu cm^{-1}$ : 3430 and 3380 (duplet, s), 3300 (s), 3220 (s), 1660 and 1635 (duplet, s), 1600 (s) and 1535 (s), (143.0 g) is suspended in methanol (2000 ml) and keeping the internal temperature between 20-22°C, concentrated aqueous hydrochloric acid (35.0%, 85.0 ml) is added. To the resulting solution, bicyclic amidine DBU (147.5 g) is added all at once, reaching about 35°C. After some time (10-15 min., aproximately), it becomes turbid. Immediately it is heated to reflux temperature, obtaining a colorless solution at temperature of 50-55°C. It is diluted with chloroform (5000 ml), and at about 35-36°C precipitation begins. After cooling at 10-15°C for three hours, it is filtered, washed with methanol, with dichloromethane, and dried, resulting 107.2 g of the title compound, m.p. 159-161°C, bulk density = 0.20, infrared spectrum and crystals identical to those of Figure 1 and Figure 3, respectively.

Observation with a stereoscopic microscope (Kiowa SDZ-PL, model provided with Zoom), shows a homogeneous mixture of snow white short prisms.

Recovery of solvents is realized by usual methods. By removal of the solvent from the filtrate, 25.0 g more of the title compound are obtained, which is recycled in the conversion process.

Example 4

High-density polymorph CMI-BA

Following Example 2, and substituting the triethylamine by DBN, the title compound is obtained with identical characteristics and in a similar yield.

Example 5

Low-density polymorph CMI-BB

Following Example 3, and substituting the DBU by N-methyl-morpholine, the title compound is obtained with identical characteristics and in a similar yield.

Example 6

High-density polymorph CMI-BA

Following Example 2, and adjusting properly the amounts of methanol and dichlorometane, the triethylamine is substituted by one of the following organic bases : N-ethylpiperidine, N-tripropylamine,N-tributylamine, diethylamine, dipropylamine, N-propylamine. The title compound provided with identical physicochemical characteristics and in a similar yield is obtained.

Example 7

Low-density polymorph CMI-BB

Famotidine (m.p. 167.5-169,5°C, bulk density 0.78 ; 150.0 g) suspended in methanol (2250 ml), is dissolved by addition of aqueous hydrochloric acid (35.0% ; 90 ml). Triethylamine (155.0 ml) is next added and the mass is heated at reflux, so dissolving any eventual precipitate formed. To the hot solution methylene chloride (2000 ml) is added, causing precipitation. After 4 hours (5-10°C), 113.6 g of the title compound are isolated, m.p. 157-159°C and bulk density = 0.29. Its infrared spectrum is identical to that shown in Figure 1, the crystals being like those of Figure 3.

Example 8

High-density polymorph CMI-BA

Starting from the same Famotidine of Example 7, and following the same procedure, dichloromethane (1000 ml) is added, and the resulting solution is left undisturbed (10 h.) at 0-5°C. It results 117.0 g of the title compound, m.p. 158.0-160.5°C, bulk density = 0.69. The infrared spectrum and the crystals are identical to those of Figure 1 and Figure 2, respectively.

Example 9

Low density polymorph (CMI-BB)

Famotidine (7.5 g ; m.p. : 173-176°C, physical from : concretions) is suspended n methanol (30 ml). Addition of concentrated aqueous hydrochloric acid (4.65 ml ; 35.0%) results immediately in a colorless solution. After cooling ince ice-water bath (0-5°C), triethylamine (6.25 ml) is added all at once, controlling the PH = 7.5. An abundant white precipitate is formed, and the mass is stirred (30 min.), filtered, washed with methanol and with dichloromethane. After drying, Famotidine (6.16 g), m.p. 155-161°C is obtained, which is next suspended in methanol, adjusting the pH to 7.0-7.5 with triethylamine and stirred (15 min.). By filtration a solid residue is obtained which is washed and dried, its bulk density being 0.20 to 0.22 g/ml. Microscopic observation shows it is formed by conglomerates of minute crystals, m.p. 159-161°C. The IR and UV spectra identical to those of the high-density polymorph.

Example 10

Low-density polymorph CMI-BB

Instead of the Famotidine, the starting material is Famotidine hydrochloride prepared as described in Example 1A. Operating as in the preceding example, Famotidine is obtained with identical results.

Example 11

High-density polymorph CMI-BA, by seeding

To a suspension of Famotidine (300.0 g) in methanol (4500 ml), concentrated aqueous hydrochloric acid (180 ml, 35%) is added, a solution being obtained. Next, triethylamine (310,0 ml) is added and heated to reflux obtaining a colorless solution. While still hot, chloroform (2000 ml) is added to the resulting mass and, after cooling it down to 10-15°C, it is seeded with high density polymorph. The solution is let to stand overnight at 4-5°C. The residue collected by filtration, after being washed with methanol and with dichloromethane, is Famotidine polymorph CMI-BA (255.2 g) consisting in big acicular prisms, bulk density 0.77-0.80 g/ml, m.p. 158-161°C.

Example 12

Low-density polymorph CMI-BB, by seeding

As in Example 3. The solution obtained after adding DBU (144 ml) is heated to reflux temperature and next diluted with chloroform and immediately seeded with CMI-BB. The procedure followed from here on is exactly as that of Example 3, giving the title compound with similar results.

Example 13

Low-density polymorph CMI-BB, by seeding

As in Example 2 or Example 11. The hot solution, after dilution with the chlorinated solvent, is seeded with CMI-BB. The crystals produced, correspond to the title compound, with virtually identical characteristics and yield.

**Claims**

**Claims for the following Contracting States : AT, BE, CH, DE, FR, GB, IT, LI, LU, NL, SE**

1. Famotidine polymorph CMI-BB characterized by a bulk density comprised between 0.20 and 0.30 g/ml, a melting range within 157-161°C, and the following X-Ray-diffraction data, where d is the spacing, and $I/I_1$ is the relative instensity at $\lambda$ = 1.5405 Å, using a radiation source of Cu, 40 kw and 20 ma (Siemens Kristalloflex 810 Interface DACO-MP equipment) :

| d: | $I/I_1$ |
|---|---|
| 14.68 | 0.28 |
| 7.62 | 0.58 |
| 7.15 | 0.14 |
| 5.87 | 0.12 |
| 5.61 | 0.41 |
| 5.03 | 0.20 |
| 4.92 | 0.38 |
| 4.57 | 0.41 |
| 4.42 | 1.00 |
| 4.31 | 0.20 |
| 4.25 | 0.64 |
| 3.95 | 0.52 |
| 3.89 | 0.41 |
| 3.81 | 0.32 |
| 3.69 | 0.68 |
| 3.61 | 0.33 |
| 3.47 | 0.09 |
| 3.40 | 0.19 |
| 3.37 | 0.20 |
| 3.27 | 0.21 |
| 3.18 | 0.06 |
| 3.09 | 0.08 |
| 2.95 | 0.28 |
| 2.91 | 0.12 |
| 2.85 | 0.13 |
| 2.77 | 0.36 |
| 2.72 | 0.05 |
| 2.62 | 0.06 |

| | |
|---|---|
| 2.53 ..... | 0.19 |
| 2.50 ..... | 0.14 |
| 2.46 ..... | 0.07 |
| 2.42 ..... | 0.07 |
| 2.40 ..... | 0.09 |
| 2.29 ..... | 0.10 |
| 2.23 ..... | 0.06 |
| 2.20 ..... | 0.11 |
| 2.17 ..... | 0.07 |
| 2.15 ..... | 0.12 |
| 2.12 ..... | 0.06 |
| 2.11 ..... | 0.11 |
| 2.03 ..... | 0.08 |
| 1.97 ..... | 0.07 |
| 1.85 ..... | 0.05 |
| 1.83 ..... | 0.06 |
| 1.79 ..... | 0.06 |
| 1.76 ..... | 0.06 |
| 1.74 ..... | 0.07 |
| 1.70 ..... | 0.05 |
| 1.61 ..... | 0.05 |
| 1.60 ..... | 0.13 |

the crystals being stable.

2. Famotidine CMI-BB, according to claim 1, for use as a therapeutically active substance, against diseases of the digestive track, gastric and duodenal ulcers, and the Zollinger-Ellison syndrome.

3. Pharmaceutical formulations for treatment of disease of the digestive track, gastric and duodenal ulcers, and the Zollinger-Ellison syndrome, containing Famotidine CMI-BB according to claim 1, and a pharmaceutically inert excipient.

4. Process for the preparation of Famotidine polymorph CMI-BB according to claim 1, characterized in that to a solution in methanol of Famotidine hydrochloride, hydrobromide, sulfate, nitrate or phosphate, a tertiary organic base that forms with the acid of the Famotidine salt a soluble salt in methanol or in its mixture with dichloromethane or chloroform, is quickly added, and by heating to 50-65°C a solution is obtained which is diluted with dichloromethane or chloroform, (thus causing a quick crystallization), and which is filtered at the temperature of 0-15°C.

5. Process according to claim 4, characterized in that the tertiary organic base is triethylamine or 1,8-diazabicyclo[5.4.0]undec-7-ene.

6. Process according to claim 4, characterized in that the solution in methanol of Famotidine hydrochloride, is prepared from Famotidine polymorph of either high melting point or high bulk density, or its mixtures, and hydrogen chloride, that the tertiary organic base is 1,8-diazabicyclo [5.4.0]undec-7-ene and, that after dilution of the solution with dichloromethane or chloroform, a seed of Famotidine polymorph CMI-BB is added.

7. Famotidine polymorph CMI-BB of claim 1 for use in pharmaceutical formulations.

**Claims for the following Contracting States : ES, GR**

1. Process for the preparation of Famotidine polymorph CMI-BB, having a bulk density comprised between 0,20 and 0,30 g/ml, a melting range within 157-161°C and exhibiting the following X-Ray-diffraction data, where

d is the spacing and $I/I_1$ is the relative instensity at $\lambda$ = 1.5405 Å, using a radiation source of Cu, 40 kw and 20 ma (Siemens Kristalloflex 810 Interface DACO-MP equipment) :

| d: | $I/I_1$ |
|---|---|
| 14.68 | 0.28 |
| 7.62 | 0.58 |
| 7.15 | 0.14 |
| 5.87 | 0.12 |
| 5.61 | 0.41 |
| 5.03 | 0.20 |
| 4.92 | 0.38 |
| 4.57 | 0.41 |
| 4.42 | 1.00 |
| 4.31 | 0.20 |
| 4.25 | 0.64 |
| 3.95 | 0.52 |
| 3.89 | 0.41 |
| 3.81 | 0.32 |
| 3.69 | 0.68 |
| 3.61 | 0.33 |
| 3.47 | 0.09 |
| 3.40 | 0.19 |
| 3.37 | 0.20 |
| 3.27 | 0.21 |
| 3.18 | 0.06 |
| 3.09 | 0.08 |
| 2.95 | 0.28 |
| 2.91 | 0.12 |
| 2.85 | 0.13 |
| 2.77 | 0.36 |
| 2.72 | 0.05 |
| 2.62 | 0.06 |

| | |
|---|---|
| 2.53 ..... | 0.19 |
| 2.50 ..... | 0.14 |
| 2.46 ..... | 0.07 |
| 2.42 ..... | 0.07 |
| 2.40 ..... | 0.09 |
| 2.29 ..... | 0.10 |
| 2.23 ..... | 0.06 |
| 2.20 ..... | 0.11 |
| 2.17 ..... | 0.07 |
| 2.15 ..... | 0.12 |
| 2.12 ..... | 0.06 |
| 2.11 ..... | 0.11 |
| 2.03 ..... | 0.08 |
| 1.97 ..... | 0.07 |
| 1.85 ..... | 0.05 |
| 1.83 ..... | 0.06 |
| 1.79 ..... | 0.06 |
| 1.76 ..... | 0.06 |
| 1.74 ..... | 0.07 |
| 1.70 ..... | 0.05 |
| 1.61 ..... | 0.05 |
| 1.60 ..... | 0.13 |

characterized in that to a solution in methanol of Famotidine hydrochloride, hydrobromide, sulfate, nitrate or phosphate, a tertiary organic base that forms with the acid of the famotidine salt a soluble salt in methanol or in its mixture with dichloromethane or chloroform, is quickly added, and by heating to 50-65°C a solution is obtained which is diluted with dichloromethane or chloroform, (thus causing a quick crystallization), and which is filtered at a temperature of 0-15°C.

2. Process according to claim 1, characterized in that the tertiary organic base is triethylamine or 1,8-diazabicyclo[5.4.0]unde-7-ene.

3. Process according to claim 1, characterised in that the solution in methanol of the Famotidine hydrochloride is prepared from Famotidine polymorph of either high melting or high bulk density, or its mixtures and hydrogen chloride, that the tertiary organic base is 1,8-diazabicyclo[5.4.0]undec-7-ene and that after dilution of the solution with dichloromethane or chloroform, a seed of Famotidine polymorph CMI-BB is added.

4. Process according to claim 1 for the preparation, of the Famotidine polymorph as defined in claim 1 for use in pharmaceutical formulations.

## Patentansprüche

### Patentanprüche für folgende Vertragsstaaten : AT, BE, CH, DE, FR, GB, IT, LI, LU, NL, SE

1. Famotidin Polymorph CMI-BB, gekennzeichnet durch eine Schüttdichte zwischen 0,20 und 0,30 g/ml, einen Schmelzbereich zwischen 157-161°C und die nachstehend genannten Röntgenbeugungswerte, bei denen d der Abstand und $I/I_1$ die relative Intensität $\lambda$ = 1,5405 Å unter Benutzung einer Kupfer-Röntgenquelle mit 40 kW und 20 mA (Siemens Kristalloflex 810 Interface DACO-MP) ist :

| d: | I/I₁ |
|---|---|
| 14.68 | 0.28 |
| 7.62 | 0.58 |
| 7.15 | 0.14 |
| 5.87 | 0.12 |
| 5.61 | 0.41 |
| 5.03 | 0.20 |
| 4.92 | 0.38 |
| 4.57 | 0.41 |
| 4.42 | 1.00 |
| 4.31 | 0.20 |
| 4.25 | 0.64 |
| 3.95 | 0.52 |
| 3.89 | 0.41 |
| 3.81 | 0.32 |
| 3.69 | 0.68 |
| 3.61 | 0.33 |
| 3.47 | 0.09 |
| 3.40 | 0.19 |
| 3.37 | 0.20 |
| 3.27 | 0.21 |
| 3.18 | 0.06 |
| 3.09 | 0.08 |
| 2.95 | 0.28 |

| | |
|---|---|
| 2.91 ..... | 0.12 |
| 2.85 ..... | 0.13 |
| 2.77 ..... | 0.36 |
| 2.72 ..... | 0.05 |
| 2.62 ..... | 0.06 |
| 2.53 ..... | 0.19 |
| 2.50 ..... | 0.14 |
| 2.46 ..... | 0.07 |
| 2.42 ..... | 0.07 |
| 2.40 ..... | 0.09 |
| 2.29 ..... | 0.10 |
| 2.23 ..... | 0.06 |
| 2.20 ..... | 0.11 |
| 2.17 ..... | 0.07 |
| 2.15 ..... | 0.12 |
| 2.12 ..... | 0.06 |
| 2.11 ..... | 0.11 |
| 2.03 ..... | 0.08 |
| 1.97 ..... | 0.07 |
| 1.85 ..... | 0.05 |
| 1.83 ..... | 0.06 |
| 1.79 ..... | 0.06 |
| 1.76 ..... | 0.06 |
| 1.74 ..... | 0.07 |
| 1.70 ..... | 0.05 |
| 1.61 ..... | 0.05 |
| 1.60 ..... | 0.13 |

wobei die Kristalle stabil bleiben.

2. Famotidin CMI-BB nach Anspruch 1, zur Verwendung als therapeutisch wirksame Substanz bei der Behandlung von Erkrankungen des Verdauungstrakts, von Magen- und Zwölffingerdarmgeschwüren und des Zollinger-Ellison-Syndroms.

3. Pharmazeutische Zubereitungen zur Behandlung von Erkrankungen des Verdauungstrakts, von Magen- und Zwölffingerdarmgeschwüren und des Zollinger-Ellison-Syndroms, die Famotidin CMI-BB nach Anspruch 1 und ein pharmazeutisch wirkungsloses Vehikel enthalten.

4. Verfahren zur Herstellung von Famotidin Polymorph CMI-BB nach Anspruch 1, dadurch gekennzeichnet, dass eine tertiäre organische Base, die mit der Säure des Famotidinsalzes ein in Methanol oder in dessen Mischung mit Dichlormethan oder Chloroform lösliches Salz bildet, rasch zu in Methanol gelöstem Famotidinhydrochlorid, -hydrobomid, -sulfat, -nitrat oder -phosphat hinzugegeben wird, wobei sich durch Erwärmung auf 50-65°C eine Lösung ergibt, die (für eine schnelle Kristallisation) mit Dichlormethan oder Chloroform verdünnt und anschliessend bei einer Temperatur von 0-15°C abgefiltert wird.

5. Verfahren nach Anspruch 4, dadurch gekennzeichnet, dass als tertiäre organische Base Triäthylamin oder 1,8-Diazabicyclo[5.4.0]undec-7-en Verwendung findet.

6. Verfahren nach Anspruch 4, dadurch gekennzeichnet, dass die Methanollösung von Famotidinhyddrochlorid aus Famotidin Polymorph mit einem hohen Schmelzpunkt oder einer hohen Schüttdichte oder deren

Mischungen und Wasserstoffchlorid hergestellt wird, dass als tertiäre organische Base 1,8-Diazabicyclo[5.4.0]undec-7-en Verwendung findet, und dass nach Verdünnung der Lösung mit Dichlormethan oder Chloroform ein Keim Famotidin Polymorph CMI-BB zugegeben wird.

7. Famotidin Polymorph CMI-BB nach Anspruch 1 zur Verwendung bei pharmazeutischen Zubereitungen.

**Patentansprüche für folgende Vertragsstaaten : ES, GR**

1. Verfahren zur Herstellung von Famotidin Polymorph CMI-BB mit einer Schüttdichte zwischen 0,20 und 0,30 g/ml, einem Schmelzbereich zwischen 157-161°C und den nachstehend genannten Röntgenbeugungswerten, bei denen d der Abstand und $I/I_1$ die relative Intensität bei $\lambda = 1{,}5405$ Å unter Benutzung einer Kupfer-Röntgenquelle mit 40 kW und 20 mA (Siemens Kristalloflex 810 Interface DACO-MP) ist :

| d: | | $I/I_1$ |
|---|---|---|
| 14.68 | ..... | 0.28 |
| 7.62 | ...... | 0.58 |
| 7.15 | ..... | 0.14 |
| 5.87 | ..... | 0.12 |
| 5.61 | ..... | 0.41 |
| 5.03 | ..... | 0.20 |
| 4.92 | ..... | 0.38 |
| 4.57 | ..... | 0.41 |
| 4.42 | ..... | 1.00 |
| 4.31 | ..... | 0.20 |
| 4.25 | ..... | 0.64 |
| 3.95 | ..... | 0.52 |
| 3.89 | ..... | 0.41 |
| 3.81 | ..... | 0.32 |
| 3.69 | ..... | 0.68 |
| 3.61 | ..... | 0.33 |
| 3.47 | ..... | 0.09 |
| 3.40 | ..... | 0.19 |
| 3.37 | ..... | 0.20 |
| 3.27 | ..... | 0.21 |
| 3.18 | ..... | 0.06 |
| 3.09 | ..... | 0.08 |
| 2.95 | ..... | 0.28 |
| 2.91 | ..... | 0.12 |

| | |
|---|---|
| 2.85 | 0.13 |
| 2.77 | 0.36 |
| 2.72 | 0.05 |
| 2.62 | 0.06 |
| 2.53 | 0.19 |
| 2.50 | 0.14 |
| 2.46 | 0.07 |
| 2.42 | 0.07 |
| 2.40 | 0.09 |
| 2.29 | 0.10 |
| 2.23 | 0.06 |
| 2.20 | 0.11 |
| 2.17 | 0.07 |
| 2.15 | 0.12 |
| 2.12 | 0.06 |
| 2.11 | 0.11 |
| 2.03 | 0.08 |
| 1.97 | 0.07 |
| 1.85 | 0.05 |
| 1.83 | 0.06 |
| 1.79 | 0.06 |
| 1.76 | 0.06 |
| 1.74 | 0.07 |
| 1.70 | 0.05 |
| 1.61 | 0.05 |
| 1.60 | 0.13 |

dadurch gekennzeichnet, dass eine tertiäre organische Base, die mit der Säure des Famotidinsalzes ein in Methanol oder in dessen Mischung mit Dichlormethan oder Chloroform lösliches Salz bildet, rasch zu in Methanol gelöstem Famotidinhydrochlorid, -hydrobromid, -sulfat, -nitrat oder -phosphat hinzugegeben wird, wobei sich durch Erwärmung auf 50-65°C eine Lösung ergibt, die (für eine schnelle Kristallisation) mit Dichlormethan oder Chloroform verdünnt und anschliessend bei einer Temperatur von 0-15°C abgefiltert wird.

2. Verfahren nach Anspruch 1, dadurch gekennzeichnet, dass als tertiäre organische Base Triäthylamin oder 1,8-Diazabicyclo[5.4.0]undec-7-en Verwendung findet.

3. Verfahren nach Anspruch 1, dadurch gekennzeichnet, dass die Methanollösung von Famotidinhydrochlorid aus Famotidin Polymorph mit einem hohen Schmelzpunkt oder einer hohen Schüttdichte oder deren Mischungen und Wasserstoffchlorid hergestellt wird, dass als tertiäre organische Base 1,8-Diazabicyclo[5.4.0]undec-7-en Verwendung findet, und dass nach Verdünnung der Lösung mit Dichlormethan oder Chloroform ein Keim Famotidin Polymorph CMI-BB zugegeben wird.

4. Verfahren nach Anspruch 1 zur Herstellung von Famotidin Polymorph nach der Definition von Anspruch 1 zur Verwendung bei pharmazeutischen Zubereitungen.

## Revendications

### Revendications pour les Etats contractants suivants : AT, BE, CH, DE, FR, GB, IT, LI, LU, NL, SE

1. Famotidine polymorphe CMI-BB caractérisée par une densité de masse comprise entre 0,20 et 0,30 g/ml, une échelle de fusion allant de 157 à 161°C, et les données suivantes sur la diffraction des rayons X, où d est l'intervalle, et $I/I_1$ est l'intensité relative à $\lambda$ = 1,5405 Å, utilisant une source de rayonnement de Cu, 40 kW et 20 mA (équipement Siemens Kristalloflex 810 Interface DACO-MP) :

| d: | | $I/I_1$ |
|---|---|---|
| 14.68 | ..... | 0.28 |
| 7.62 | ...... | 0.58 |
| 7.15 | ..... | 0.14 |
| 5.87 | ..... | 0.12 |
| 5.61 | ..... | 0.41 |
| 5.03 | ..... | 0.20 |
| 4.92 | ..... | 0.38 |
| 4.57 | ..... | 0.41 |
| 4.42 | ..... | 1.00 |
| 4.31 | ..... | 0.20 |
| 4.25 | ..... | 0.64 |
| 3.95 | ..... | 0.52 |
| 3.89 | ..... | 0.41 |
| 3.81 | ..... | 0.32 |
| 3.69 | ..... | 0.68 |
| 3.61 | ..... | 0.33 |
| 3.47 | ..... | 0.09 |
| 3.40 | ..... | 0.19 |
| 3.37 | ..... | 0.20 |
| 3.27 | ..... | 0.21 |
| 3.18 | ..... | 0.06 |
| 3.09 | ..... | 0.08 |
| 2.95 | ..... | 0.28 |

| | |
|---|---|
| 2.91 ..... | 0.12 |
| 2.85 ..... | 0.13 |
| 2.77 ..... | 0.36 |
| 2.72 ..... | 0.05 |
| 2.62 ..... | 0.06 |
| 2.53 ..... | 0.19 |
| 2.50 ..... | 0.14 |
| 2.46 ..... | 0.07 |
| 2.42 ..... | 0.07 |
| 2.40 ..... | 0.09 |
| 2.29 ..... | 0.10 |
| 2.23 ..... | 0.06 |
| 2.20 ..... | 0.11 |
| 2.17 ..... | 0.07 |
| 2.15 ..... | 0.12 |
| 2.12 ..... | 0.06 |
| 2.11 ..... | 0.11 |
| 2.03 ..... | 0.08 |
| 1.97 ..... | 0.07 |
| 1.85 ..... | 0.05 |
| 1.83 ..... | 0.06 |
| 1.79 ..... | 0.06 |
| 1.76 ..... | 0.06 |
| 1.74 ..... | 0.07 |
| 1.70 ..... | 0.05 |
| 1.61 ..... | 0.05 |
| 1.60 ..... | 0.13 |

les cristaux étant stables.

2. Famotidine CMI-BB, conformément à la revendication 1, pour son utilisation en tant que substance thérapeutique active, contre des maladies de l'appareil digestif, ulcères de l'estomac et du duodénum, et le syndrome de Zollinger-Ellison.

3. Formulations pharmaceutiques pour le traitement de maladies de l'appareil digestif, ulcères de l'estomac et du duodénum, et le syndrome de Zollinger-Ellison, contenant de la Famotidine CMI-BB conformément à la revendication 1, et un excipient inerte d'un point de vue pharmaceutique.

4. Procédé pour la préparation de la Famotidine polymorphe CMI-BB conformément à la revendication 1, qui se caractérise en ce que, à une solution dans le méthanol d'hydrochlorure de Famotodine, hydrobromure, sulfate, nitrate ou phosphate, on ajoute rapidement une base organique tertiaire qui forme avec l'acide du sel de Famotidine un sel soluble dans le méthanol ou son mélange avec du dichlorométhane ou du chloroforme, et en chauffant à 50-65°C on obtient une solution qui est diluée avec du dichlorométhane ou du chloroforme (provoquant ainsi une rapide cristallisation), et qui est filtrée à une température de 0-15°C.

5. Procédé conformément à la revendication 4, qui se caractérise en ce que la base organique tertiaire est la triéthylamine ou 1,8-diazabicyclo[5.4.0]undec-7-ène.

6. Procédé conformément à la revendication 4, qui se caractérise en ce que la solution dans le méthanol d'hydrochlorure de Famotidine est préparée à partir de Famoticidine polymorphe soit avec un point de fusion

élevé soit avec une densité de masse élevée, ou ses mélanges, et du chlorure d'hydrogène, que la base organique tertiaire est 1,8-diazabicyclo[5.4.0]undec-7-ène, et qu'après la dilution de la solution avec du dichlorométhane ou de chloroforme, on ajoute un germe de Famotidine polymorphe CMI-BB.

7. Famotidine polymorphe CMI-BB de la revendication 1 pour usage dans des formulations pharmaceutiques.

**Revendications pour les Etats contractants suivants : ES, GR**

1. Procédé pour la préparation de Famotidine polymorphe CMI-BB, ayant une densité de masse comprise entre 0,20 et 0,30 g/ml, une échelle de fusion allant de 157 à 161°C et présentant les données suivantes sur la diffraction des rayons X, où d est l'intervalle, et $I/I_1$ est l'intensité relative à $\lambda$ = 1,5405 Å, utilisant une source de rayonnement de Cu, 40 kW et 20 mA (équipement Siemens Kristalloflex 810 Interface DACO-MP) :

| d: | $I/I_1$ |
|---|---|
| 14.68 | 0.28 |
| 7.62 | 0.58 |
| 7.15 | 0.14 |
| 5.87 | 0.12 |
| 5.61 | 0.41 |
| 5.03 | 0.20 |
| 4.92 | 0.38 |
| 4.57 | 0.41 |
| 4.42 | 1.00 |
| 4.31 | 0.20 |
| 4.25 | 0.64 |
| 3.95 | 0.52 |
| 3.89 | 0.41 |
| 3.81 | 0.32 |
| 3.69 | 0.68 |
| 3.61 | 0.33 |
| 3.47 | 0.09 |
| 3.40 | 0.19 |
| 3.37 | 0.20 |
| 3.27 | 0.21 |
| 3.18 | 0.06 |
| 3.09 | 0.08 |
| 2.95 | 0.28 |

| | |
|---|---|
| 2.91 | 0.12 |
| 2.85 | 0.13 |
| 2.77 | 0.36 |
| 2.72 | 0.05 |
| 2.62 | 0.06 |
| 2.53 | 0.19 |
| 2.50 | 0.14 |
| 2.46 | 0.07 |
| 2.42 | 0.07 |
| 2.40 | 0.09 |
| 2.29 | 0.10 |
| 2.23 | 0.06 |
| 2.20 | 0.11 |
| 2.17 | 0.07 |
| 2.15 | 0.12 |
| 2.12 | 0.06 |
| 2.11 | 0.11 |
| 2.03 | 0.08 |
| 1.97 | 0.07 |
| 1.85 | 0.05 |
| 1.83 | 0.06 |
| 1.79 | 0.06 |
| 1.76 | 0.06 |
| 1.74 | 0.07 |
| 1.70 | 0.05 |
| 1.61 | 0.05 |
| 1.60 | 0.13 |

qui se caractérise en ce que, à une solution dans le méthanol d'hydrochlorure de Famotidine, hydrobromure, sulfate, nitrate ou phosphate, on ajoute rapidement une base organique tertiaire qui forme avec l'acide de sel de Famotidine un sel soluble dans le méthanol ou ses mélanges avec du dichlorométhane ou de chloroforme, et en chauffant à 50-65°C, on obtient une solution qui est diluée avec du dichlorométhane ou du chloroforme (provoquant ainsi une cristallisation rapide) et qui est filtrée à une température de 0-15°C.

2. Procédé conformément à la revendication 1, qui se caractérise en ce que la base organique tertiaire est la triéthylamine ou 1,8-diazabicyclo[5.4.0]undec-7-ène.

3. Procédé conformément à la revendication 1, qui se caractérise en ce que la solution dans le méthanol d'hydrochlorure de Famotidine est préparée à partir de Famotidine polymorphe soit avec un point de fusion élevé soit avec une densité de masse élevée, ou ses mélanges, et du chlorure d'hydrogène, que la base organique tertiaire est 1,8-diazabicyclo[5.4.0]undec-7-ène, et qu'après la dilution de la solution avec du dichlorométhane ou du chloroforme, on ajoute un germe de Famoticidine polymorphe CMI-BB.

4. Procédé conformément à la revendication 1 pour la préparation de la Famoticidine polymorphe comme défini dans la revendication 1 pour usage dans des formulations pharmaceutiques.

FIG. 1

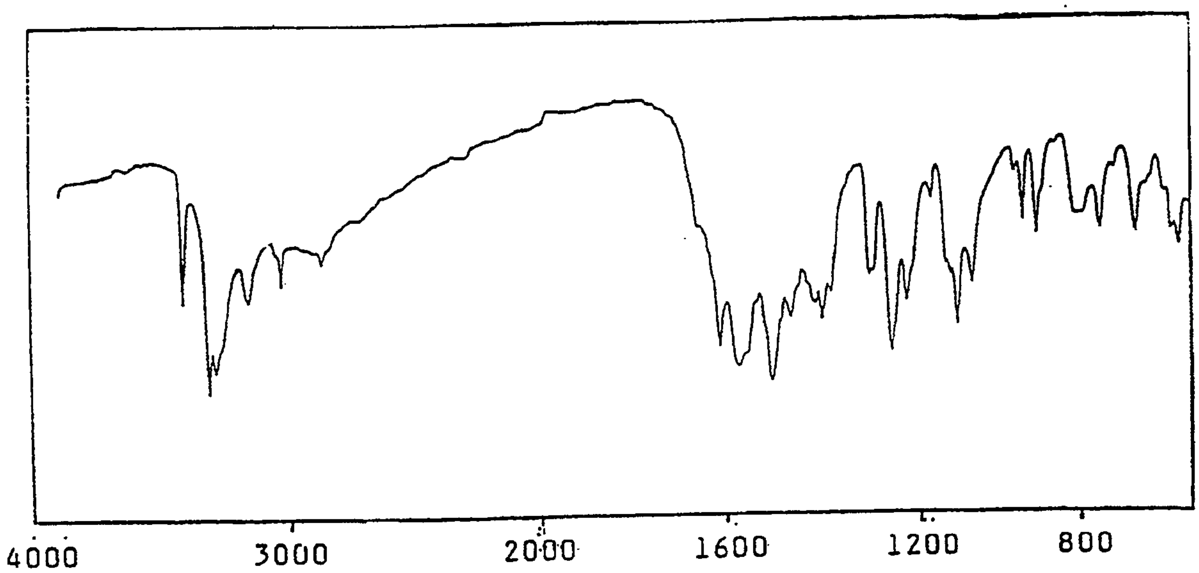


FIG. 2

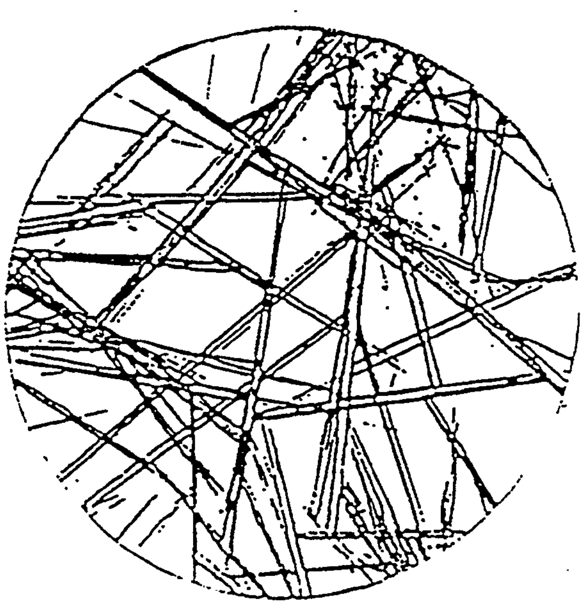

FIG. 3